# EUROPÄISCHE PATENTSCHRIFT

(11) **EP 2 172 339 B1**
(45) Veröffentlichungstag und Bekanntmachung des Hinweises auf die Patenterteilung: **28.05.2014**
(21) Anmeldenummer: 09011698.9
(22) Anmeldetag: 12.09.2009
(51) Int. Cl.: B32B 27/40, C02F 3/28, C02F 11/04

(54) **Membranfolie für Biogasanlangen**
Membrane film for biogas assemblies
Film à membrane pour installations de biogaz

(30) Priorität: 25.09.2008 DE 102008048898
(43) Veröffentlichungstag der Anmeldung: 07.04.2010
(73) Patentinhaber: Epurex Films GmbH & Co. KG, 29656 Walsrode (DE)
(72) Erfinder: Hargarter, Nicole, 24937 Flensburg (DE); Kliewer, Uwe, 25474 Bönningstedt (DE); Kosthorst, Helge, 29664 Walsrode (DE); Stenbeck, Wolfgang, 29699 Bomlitz (DE)
(74) Vertreter: Feldhues, Michael L.F.

(56) Entgegenhaltungen:
- EP-A1- 0 571 868
- EP-A1- 1 884 358
- EP-A1- 2 031 293
- EP-A2- 1 197 481
- DE-A1- 19 504 012
- US-A1- 2005 048 236
- US-A1- 2005 130 768
- US-A1- 2006 084 336

## Beschreibung

Diese Erfindung betrifft die Verwendung einer mehrschichtigen Folie mit mindestens zwei außen liegenden Schichten aus thermoplastischem Polyurethan (TPU) und mindestens einer innen liegenden Schicht auf Basis von Polyethylenvinylalkohol-Copolymer als Trennfolie zwischen Gasgemischen unterschiedlicher Zusammensetzung, insbesondere als gasdichte Membranfolie für Biogasanlagen. In einer bevorzugten Ausführung besitzt die Folie mindestens eine Schicht eines Ethylenvinylalkohol-Copolymers, um eine besonders hohe Gasdichtigkeit zu erzielen.

Aufgrund der Kombination von hohem mechanischen Eigenschaftsprofil, guter Abriebbeständigkeit, Schweißbarkeit, guter Beständigkeit gegen Mikrobenangriff, Elastizität und Flexibilität in der Kälte mit geringer Durchlässigkeit für Methan eignet sich eine TPU-Folie in hervorragender Weise für die Verwendung als Membranfolie für Biogasanlagen.

Umfangreiche Informationen zum Stand der Technik bei der Biogaserzeugung bieten z.B. die Fachagentur Nachwachsende Rohstoffe e.V. (FNR): Handreichung Biogasgewinnung und - nutzung, ISBN 3-00-014333-5 und Bayerisches Staatsministerium für Umwelt, Gesundheit und Verbraucherschutz (StMUGV): Biogas Handbuch Bayern. München, 15. November 2004.

Eine Biogasanlage dient zur Erzeugung von Biogas aus Biomasse. Als Nebenprodukt wird meistens Wärme oder Dünger (Gülle) produziert. Es werden verschiedene Rohstoffe, z.B. Bioabfall, Gülle, Klärschlamm, Fette oder Pflanzen in einen luftdicht verschlossenen Fermenter eingebracht. Dort entsteht durch anaerobe Gär- oder Fäulnisprozesse das Biogas, das je nach Ausgangsstoff aus 40-75 Vol.-% Methan, 25-55 Vol.-% Kohlendioxid, bis zu 10 Vol.-% Wasserdampf sowie darüber hinaus aus geringen Anteilen Stickstoff, Sauerstoff, Wasserstoff, Ammoniak und Schwefelwasserstoff besteht.

Häufig sind Biogasanlagen zur Steigerung der Gasausbeute zweistufig ausgeführt, d.h. dass neben dem Fermenter ein Nachgärer vorgesehen ist.

Die Biomasse wird in einem Lagerbehälter vorrätig gehalten.

Die erfindungsgemäße Folie eignet sich als Membranfolie für alle Behälterkomponenten - Fermenter, Nachgärer, Lagerbehälter - einer Biogasanlage. Die Membranfolie erfüllt dabei die Aufgabe, den offenen Behälter nach oben gasdicht abzuschließen und durch Auf- und Abbewegung einen je nach anfallender und verbrauchter Biogasmenge variablen Gasspeicherraum zu bilden. Gegen Witterungseinflüsse wird sie mit Hilfe einer Wetterschutzfolie geschützt.

Derzeit wird Biogas vor allem zur dezentralen gekoppelten Strom- und Wärmeerzeugung in Blockheizkraftwerken genutzt (Kraft-Wärme-Kopplung). Dazu wird das Gasgemisch getrocknet (der Wasseranteil im Biogas wird reduziert), durch Einblasen einer kleinen Menge Frischluft entschwefelt und dann einem Verbrennungsmotor zugeführt, der einen Generator antreibt. Der so produzierte Strom wird ins Netz eingespeist.

Die Ausgangsstoffe für die technische Produktion von Biogas sind, abgesehen von Transport-und sonstigen Nebenkosten, prinzipiell kostenlos, Stoffe wie Klärschlamm, Bioabfall, Gülle, Mist oder bisher nicht genutzte Pflanzen bzw. Pflanzenteile (z. B. Zwischenfrüchte und Kleegras im Biolandbau), oder zumindest nachwachsende Rohstoffe (gezielt angebaute Energiepflanzen).

Aufgrund der Unabhängigkeit von Wind oder Sonneneinstrahlung trägt die Biomasse und damit auch das Biogas sinnvoll dazu bei, eine Ergänzung im Energiemix der erneuerbaren Energieträger einzunehmen. Wertvoll im Biogas ist das Methan. Je höher dessen Anteil ist, desto energiereicher ist das Gas. Nicht nutzbar sind das Kohlendioxid und der Wasserdampf.

Nach Kohlendioxid ist Methan das bedeutendste von Menschen freigesetzte Treibhausgas, wobei es 20 bis 30 mal wirkungsvoller ist, d.h. das Erwärmungspotenzial von 1 kg Methan ist, auf einen Zeitraum von 100 Jahren betrachtet, ca. 25 mal höher als das von 1 kg Kohlendioxid. Quelle z.B. Climate Change 2007: The Physical Science Basis. Contribution of Working Group I to the Fourth Assessment Report of the Intergovernmental Panel on Climate Change [Solomon, S., D. Qin, M. Manning, Z. Chen, M. Marquis, K.B. Averyt, M.Tignor and H.L. Miller (eds.)], Chapter 2, Table 2.14. Cambridge University Press, Cambridge, United Kingdom and New York, NY, USA.

Methan ist ein farb- und geruchloses Gas, dessen Dichte kleiner ist als die von Luft, es steigt also in die höheren Schichten der Atmosphäre auf. Dort reagiert es mit Sauerstoff zu Kohlendioxid und Wasser. Dieser Prozess verläuft allerdings langsam, die Halbwertszeit wird auf 14 Jahre geschätzt.

Methan ist hochentzündlich, der Flammpunkt liegt bei -188 °C, die Zündtemperatur bei 600 °C. Es sollten daher bei der Handhabung Maßnahmen gegen elektrostatische Aufladung getroffen werden.

Im Kyoto-Protokoll wurde ein völkerrechtlich verbindliches Abkommen zur Reduzierung des anthropogenen Ausstoßes von wichtigen Treibhausgasen (direkte Treibhausgase) beschlossen. Die im Kyoto-Protokoll reglementierten Gase sind: Kohlendioxid (CO₂, dient als Referenzwert), Methan (CH₄), Distickstoffmonoxid (Lachgas, N₂O), teilhalogenierte und perfluorierte Fluorkohlenwasserstoffe (H-FKW/HFCs) und Schwefelhexafluorid (SF₆).

Zusätzlich existieren Minderungsziele für Ammoniakemissionen, die aus internationalen Vereinbarungen resultieren (NEC-Richtlinie), da das stechend riechend und giftige Ammoniak sich über unterschiedliche Wege schädlich auf Mensch und Natur auswirkt. In umgewandelter Form kann es auch als klimawirksames Lachgas in die Atmosphäre entweichen. Die Ammoniakemissionen stammen fast ausschließlich aus der Landwirtschaft. Da der Einsatz von Energiepflanzen wie Mais zur Biogasproduktion im Gegensatz zur Güllevergärung in jedem Fall zusätzliche Ammoniakemissionen verursacht, ist durch gasdicht abgedeckte Gärrestlager mit Restgasnutzung sowie durch optimierte Ausbringungsmethoden der Gärreste in der Landwirtschaft diesem negativen Effekt entgegenzusteuern. Nachzulesen z.B. in der Broschüre "Bio-gas und Umwelt - Ein Überblick", Stand: Juni 2008, Bundesministerium für Umwelt, Naturschutz und Reaktorsicherheit, Download über www.bmu.de.

Dort wird auch darauf hingewiesen, dass noch ein erhebliches Optimierungspotential bezüglich Geruchsemissionen aus der Biogasproduktion besteht.

Es ist bekannt, dass Folien auf Basis von Polyethylen als Membranfolien für Biogasanlagen eingesetzt werden können. Polyethylen besitzt jedoch eine relativ hohe Permeationsrate für Methan und Ammoniak und eine vergleichsweise geringe mechanische Festigkeit und Kälteflexibilität.

Einschichtige Folien aus thermoplastischen Polyurethanen (TPU), Verfahren zu ihrer Herstellung sowie ihre Verwendung sind beispielsweise aus der EP-A 0 308 683, der EP-A 0 526 858, der EP-A 0 571 868 oder der EP-A 0 603 680 bekannt. Die in diesen Schriften beschriebenen Aufbauten lassen sich auch in mehrschichtige Folien integrieren.

Die Verwendung von Ethylenvinylalkohol-Copolymeren als gasdichte Schicht in mehrschichtigen Kunststofffolien für Lebensmittelverpackungen ist ebenfalls Stand der Technik. Diese Folien bestehen aus Polymeren mit hohem Weg/Spannungs-Modul und eignen sich aufgrund ihrer hohen Steifigkeit nicht für die Verwendung als Membranfolie für Biogasanlagen.

Es stellte sich daher die Aufgabe, eine flexible, elastische Folie für die Verwendung als Membranfolie für Biogasanlagen bereitzustellen, die neben einer geringen Durchlässigkeit für Methan ein mechanisches Eigenschaftsprofil aufweisen sollte, das dem von Polyethylen überlegen ist. Zusätzlich sollte sie schweißbar, kälteflexibel und beständig gegen die Medieneinflüsse im Gasraum sein. Vorzugsweise sollte die Folie antistatisch ausrüstbar bzw. ausgerüstet sein. Eine geringe Durchlässigkeit für Ammoniak stellte eine weitere Anforderung dar. Gekoppelt an ihre geringe Gasdurchlässigkeit sollte die Folie auch Geruchsemissionen verringern.

Diese Aufgabe wird erfindungsgemäß durch die Bereitstellung einer Membranfolie gelöst, die den genannten Anforderungen an eine für die Verwendung in Biogasanlagen geeigneten Folie genügt. Diese erfindungsgemäße Membranfolie ist dadurch gekennzeichnet, dass sie mehrschichtig aufgebaut ist, wobei mindestens zwei außen liegende Schichten aus thermoplastischen Polyurethanen (TPU) bestehen und eine Schicht auf Basis von Polyethylenvinylalkohol-Copolymer innen liegt.

Erfindungsgemäß bevorzugt werden TPU eingesetzt, deren längerkettige Diolkomponente ein Polyester oder Polyether ist, und die eine Shore-Härte von vorzugsweise 75 - 95 A, besonders bevorzugt 85 - 95 A, bestimmt nach DIN 53 505, aufweisen. Besonders bevorzugt werden TPU mit Polyether-Diolkomponente (TPU-Ether).

Eine weitere erfindungsgemäße Ausführung stellt die Kombination von TPU-Ether- und TPU-Ester-Schichten in einer Mehrschichtfolie und ihre erfindungsgemäße Verwendung dar.

Geeignete thermoplastische Polyurethane sind beispielsweise unter den Handelsnamen Desmopan^{®} und Texin^{®} (Bayer MaterialScience AG, Leverkusen, DE), Elastollan^{®} (Elastogran GmbH, Lemförde, DE), Estane^{®} (Lubrizol Advanced Materials Inc., Cleveland, OH, USA) oder Morthane^{®} (Morton International, Inc., Chicago, Ill, USA) erhältlich.

Eine anwendungsgeeignete Ausführung der erfindungsgemäßen Folien kann in der aus thermoplastischen Polyurethanen gebildeten Schicht zusätzlich gebräuchliche Additive enthalten, die aus der Gruppe umfassend
I. Antiblockmittel, anorganische oder organische Abstandshalter,
II. Gleit- oder Entformungsmittel,
III. Pigmente oder Füllstoffe und
IV. Stabilisatoren
ausgewählt werden können.

Der Anteil der genannten Additive I bis IV liegt in Summe bevorzugt zwischen 0 Gew.-% und 30 Gew.-%.

In einer besonders bevorzugten Ausführung enthält die TPU-Schicht bis zu 30 Gew.-% eines Antistatikums.

Additive, die in den erfindungsgemäßen Folien enthalten sein können, sind beispielsweise bei Gächter und Müller beschrieben in: Plastics Additives, Carl Hanser Verlag München, 4. Ausgabe (1996).

Ein mindestens dreischichtiger Aufbau einer Mehrschichtfolie und ihre erfindungsgemäße Verwendung sind **dadurch gekennzeichnet, dass** mindestens eine Schicht auf Basis eines Ethylenvinylalkohol-Copolymers zwischen zwei aus thermoplastischem Polyurethan aufgebauten Schichten liegt. Dieser Aufbau stellt eine besonders bevorzugte Ausführung der erfindungsgemäßen Folie dar.

Ethylen-Vinylalkohol-Copolymere werden in erster Linie als Sauerstoffbarriere in Lebensmittelverpackungen zur Verlängerung der Haltbarkeit und als Kohlenwasserstoffbarriere in Treibstofftanks verwendet. Ihre Eigenschaften werden über ihren Ethylen-Anteil definiert: geringere Ethylen-Anteile ergeben bessere Barriereeigenschaften, höhere Ethylen-Anteile reduzieren die erforderliche Extrusionstemperatur. Eine erfindungsgemäß bevorzugte EVOH-Schicht hat einen Ethylen-Gehalt zwischen 30 und 50 mol-%.

Geeignete Ethylenvinylalkohol-Copolymere sind beispielsweise unter den Handelsnamen Eval^{®} (Mitsui & Co.,LTD., Tokyo, JP) oder Soarnol^{®} (Nippon Gohsei (UK) Limited, Saltend, Kingston, UK) erhältlich.

Ein weiterer bevorzugter mindestens fünfschichtiger Aufbau sieht TPU in den beiden Außenschichten, eine Mittelschicht auf Basis eines Ethylen-Vinylalkohol-Copolymers und Zwischenschichten, sog. Haftvermittlerschichten, die aus einem Copolymer-basierenden Haftvermittler gebildet werden, vor.

Copolymer-basierende Haftvermittler sind durch Abstufungen sowohl polarer als auch unpolarer Molekülbestandteile gekennzeichnet. Besonders bevorzugt sind Olefin-basierende Polymer-Haftvermittler.

Zu der Gruppe der Olefin-basierenden Polymer-Haftvermittler gehören insbesondere EthylenVinylacetat-Copolymere und Ethylen-Acrylat-Copolymere mit einem Comonomer-Anteil niedriger als der Olefin-Anteil. Besonders bevorzugt sind Olefin-basierende Copolymere mit Comonomer-Anteilen von 15 bis 30 Gew.-%.

Für die erfindungsgemäße Verwendung bevorzugt sind Folien mit einer Gesamtdicke zwischen 50 µm und 800 µm.

Bei einer mehrschichtigen Folie liegen die Dicke der Einzelschichten aus thermoplastischen Polyurethanen bevorzugt zwischen 20 µm und 350 µm, die Dicke der Ethylen-Vinylalkohol-Schicht zwischen 5 µm und 50 µm und die der Haftvermittler-Schichten bevorzugt zwischen 5 µm und 200 µm.

In einer bevorzugten Ausführungsform der vorliegenden Erfindung besteht die Trennfolie aus mindestens zwei außen liegenden Schichten TPU und einer innen liegenden Schicht Polyethylenvinylalkohol-Copolymer. Die Methan-Permeation einer solchen Trennfolie ist kleiner als 150 cm³/m²•d•bar, vorzugsweise kleiner 1 cm³/m²•d•bar, gemessen nach ISO 15195-1 bei 23 °C/ 0 % relative Feuchte, bezogen auf eine Foliendicke von 100 µm. Die Ammoniak-Permeation einer solchen Trennfolie ist kleiner als 100 cm³/m²•d•bar gemessen nach ISO 15195-1 bei 23 °C/ 0 % relative Feuchte bezogen auf eine Foliendicke von 100 µm.

Zur Herstellung der erfindungsgemäßen Ein- oder Mehrschichtfolie eignen sich besonders die gängigen thermischen Umformverfahren zur Verarbeitung von Kunststoffen zu ein- oder mehrschichtigen Flächengebilden. Hier wäre die Herstellung durch (Co-)Extrusion zu nennen, die bevorzugt nach dem Blasfolienverfahren erfolgt. Aufgrund der besseren erzielbaren Verbundhaftung ist die Coextrusion unter den geeigneten Herstellungsverfahren von mehrschichtigen thermoplastischen Flächengebilden im besonderen Maß bevorzugt.

Die erfindungsgemäßen Folien können mit den bekannten physikalischen und chemischen Behandlungsmethoden wie beispielsweise der Corona-Behandlung ein- oder beidseitig in ihren Oberflächeneigenschaften modifiziert werden.

Um die als Bahnenware hergestellte erfindungsgemäße Folie für die erfindungsgemäße Verwendung einzusetzen, können die Bahnen oder Folienzuschnitte unter Einsatz gängiger Fügeverfahren, wie z.B. Hochfrequenzschweißen oder Heizkeilschweißen, zu den Behälteröffnungen angepassten Abschlussplanen verbunden werden.

Für den Einsatz als gasdichte Abdeckfolie für das Gärrestlager kann es vorteilhaft sein, die erfindungsgemäße Folie mit einer zusätzlichen Kunststofffolie und/oder Gewebebahn flächig zu verbinden. Als zusätzliche Folie können beispielsweise die an sich bekannten Wetterschutzfolien eingesetzt werden. Für das Verbinden eignen sich z.B. das Thermokaschieren, direkt oder unter Einsatz einer Hotmelt-Klebefolie, das Klebstoffkaschieren oder ein Beschichtungsverfahren.

Die im Rahmen der nachfolgenden Beispiele und Vergleichsbeispiele beschriebenen Folien wurden durch Blasfoliencoextrusion hergestellt. Die zum Aufschluss thermoplastischer Harze geeigneten Schneckenwerkzeuge sind in ihrem Aufbau z.B. von Wortberg, Mahlke und Effen in: Kunststoffe, 84 (1994) 1131-1138, von Pearson in: Mechanics of Polymer Processing, Elsevier Publishers, New York, 1985 oder der Fa. Davis-Standard in: Paper, Film & Foil Converter 64 (1990) S. 84 - 90 beschrieben. Werkzeuge zum Ausformen der Schmelze zu Folien sind u.a. von Michaeli in: Extrusions-Werkzeuge, Hanser Verlag, München 1991 erläutert.

### Beispiele

### Beispiel 1:

Mit Hilfe eines Einschicht-Blasfolienwerkzeuges wurde eine Folie auf Basis eines TPU-Esters der Shore A-Härte 93 hergestellt, gemessen nach DIN 53 505. Dieser 80 µm dicken Schicht wurden als Additive 4 Gew.-% anorganische Abstandshalter und 1 Gew.-% Wachse zugesetzt. Sämtliche für diese Schicht eingesetzten Komponenten wurden gemeinsam in einem Extruder aufgeschmolzen.

Der Extruder und die Ringspaltdüse wurden mit Temperaturen zwischen 160°C und 200°C betrieben. Der Düsendurchmesser betrug 130 mm. Durch Anblasen mit Luft wurde die schlauchförmige Schmelzefahne abgekühlt, anschließend flachgelegt, getrennt und aufgewickelt.

### Beispiel 2:

Mit Hilfe eines Fünfschicht-Blasfolienwerkzeuges wurde eine Folie der Dicke 80 µm hergestellt, deren mittlere, 6 µm dicke Schicht aus einem Polyethylenvinylalkohol-Copolymer mit einem PE-Anteil von 32 mol-% beidseitig durch eine jeweils 12 µm starke Schicht aus einem TPU-Ether der Shore A-Härte 87 (gemessen nach DIN 53 505) abgedeckt war. Die beiden 25 µm dicken Außenschichten waren in ihrer Zusammensetzung analog dem Beispiel 1 gewählt.

Die Extrusionseinrichtungen wurden mit Temperaturen zwischen 160°C und 200°C betrieben. Die fünf Schmelzeströme wurden in einem Fünfschicht-Blasfolienkopf mit einer Verarbeitungstemperatur von 195°C übereinander gelegt und durch eine Ringspaltdüse mit einem Durchmesser von 320 mm ausgetragen. Durch Anblasen mit Luft wurde die schlauchförmige Schmelzefahne abgekühlt, anschließend flachgelegt, getrennt und aufgewickelt.

### Beispiel 3:

Mit Hilfe eines Fünfschicht-Blasfolienwerkzeuges wurde eine Folie der Dicke 80 µm hergestellt, deren mittlere, 6 µm dicke Schicht aus einem Polyethylenvinylalkohol-Copolymer mit einem PE-Anteil von 32 mol-% beidseitig durch eine jeweils 12 µm starke Schicht aus einem Anhydrid-modifizierten Ethylenvinylacetat-Copolymer mit einem Vinylacetat-Anteil von 20 Gew.-% und einem Schmelzindex von 10,9 g/10 min (190 °C/ 2,16 kg/ ISO 1133) abgedeckt war. Die beiden 25 µm dicken Außenschichten wurden aus einem TPU-Ether der Shore A-Härte 87 (gemessen nach DIN 53 505) gebildet, denen jeweils 6 Gew.-% anorganische Abstandshalter und 1,5 Gew.-% Wachs zugesetzt wurden.

Die Extrusionseinrichtungen wurden mit Temperaturen zwischen 160°C und 200°C betrieben. Die fünf Schmelzeströme wurden in einem Fünfschicht-Blasfolienkopf mit einer Verarbeitungstemperatur von 195°C übereinandergelegt und durch eine Ringspaltdüse mit einem Durchmesser von 320 mm ausgetragen. Durch Anblasen mit Luft wurde die schlauchförmige Schmelzefahne abgekühlt, anschließend flachgelegt, getrennt und aufgewickelt.

### Vergleichsbeispiel 1:

Mit Hilfe eines Einschicht-Blasfolienwerkzeuges wurde eine Folie auf Basis eines Ethylenvinylacetat-Copolymers mit einem Vinylacetat-Anteil von 10 Gew.-% in einer Dicke von 750 µm hergestellt, der 6 Gew.-% Ruß beigemischt wurden. Sämtliche für diese Folie eingesetzten Komponenten wurden gemeinsam in einem Extruder aufgeschmolzen. Der Extruder und die Ringspaltdüse wurden mit Temperaturen zwischen 160°C und 200°C betrieben. Der Düsendurchmesser betrug 400 mm. Durch Anblasen mit Luft wurde die schlauchförmige Schmelzefahne abgekühlt, anschließend flachgelegt, getrennt und aufgewickelt.

### Bewertung der im Rahmen der Beispiele und Vergleichsbeispiele hergestellten Folien:

Die Bewertung der mechanischen Eigenschaften der Folien erfolgte nach EN ISO 527-3/5/200, die Messungen zur Methan-Permeation gemäß ISO 15105-1 bei 23 °C/ 0 % relative Feuchte. Zum Nachweis der Beständigkeit der Folien gegen Medieneinflüsse im Gasraum der Biogasanlage wurden Folienmuster 6 Monate in diesen Gasraum während des regulären Betriebes der Anlage gehängt und ihre mechanischen Eigenschaften vor und nach Lagerung verglichen.

In der nachfolgenden Tabelle sind charakteristische Daten der im Rahmen der Beispiele und des Vergleichsbeispieles hergestellten Folien wiedergegebenen. Diese zeigen deutlich, dass die in den Beispielen beschriebenen erfindungsgemäßen Folien der im Rahmen des Vergleichsbeispieles dargestellten Folie gegenüber im Vorteil sind.

So ist die Bruchspannung der erfindungsgemäßen Folien aus den Beispielen 1 bis 3 mehr als doppelt so hoch wie die der Folie aus dem Vergleichsbeispiel bei gleichzeitig dramatisch geringerer Methandurchlässigkeit.

Der Abfall der Bruchspannung nach 6 Monaten Lagerung im Gasraum einer Biogasanlage liegt für die Folien aus den Beispielen 1 bis 3 in derselben Größenordnung wie für die Vergleichsfolie. Die erfindungsgemäßen Folien besitzen also eine gute Beständigkeit gegen die Medieneinflüsse im Gasraum.

**Tabelle 1: Eigenschaften der im Rahmen der Beispiele und Vergleichsbeispiele hergestellten Folien**

| **Eigenschaft** | **Bestimmungsmethode** | **Einheit** | **Beispiel (nicht erfindungsgemäß) 1** | **Beispiel 2** | **Beispiel 3** | **Vergleichsbeispiel 1** |
|---|---|---|---|---|---|---|
| Bruchspannung | EN ISO | MPa | 70 | 55 | 55 | 25 |
| | 527-3/5/200 | | | | | |
| Methandurchlässigkeit* | ISO | cm³/ | 118 | 0,08 | 0,72 | 2025 |
| (23 °C/ 0 % r.F.) | 15195-1 | m²•d bar | | | | |
| Ammoniakdurchlässigkeit* (23 °C/ 0 % r.F.) | ISO | cm³/ | - | - | 33 | 9504 |
| | 15195-1 | m²•d bar | | | | |
| Abfall der Bruchspannung nach 6 Monaten Lagerung im Gasraum | EN ISO | % | 15 | 10 | 12 | 8 |
| | 527-3/5/200 | | | | | |

| | | | | | | |
|---|---|---|---|---|---|---|
| * umgerechnet auf eine Foliendicke von 100 µm | | | | | | |

## Patentansprüche

1. Verwendung einer mehrschichtigen Folie mit mindestens zwei außen liegenden Schichten aus thermoplastischen Polyurethanen (TPU) und einer innen liegenden Schicht auf Basis von Polyethylenvinylalkohol-Copolymer als Trennfolie zwischen Gasgemischen unterschiedlicher Zusammensetzung, **dadurch gekennzeichnet, dass** die Trennfolie in einem Coextrusionsverfahren hergestellt wurde.

2. Verwendung nach Anspruch 1, **dadurch gekennzeichnet, dass** die Trennfolie als Membranfolie in Biogasanlagen Einsatz findet.

3. Verwendung nach Anspruch 1 oder 2, **dadurch gekennzeichnet, dass** die Methan-Permeation der Trennfolie kleiner 150 cm³/m²•d•bar gemessen nach ISO 15195-1 bei 23 °C/ 0 % relative Feuchte bezogen auf eine Foliendicke von 100 µm ist.

4. Verwendung nach Anspruch 1 oder 2, **dadurch gekennzeichnet, dass** die Ammoniak-Permeation der Trennfolie kleiner 100 cm³/m²•d•bar gemessen nach ISO 15195-1 bei 23 °C/ 0 % relative Feuchte bezogen auf eine Foliendicke von 100 µm ist.

5. Verwendung nach Anspruch 3 oder 4, **dadurch gekennzeichnet, dass** sich zwischen der innen angeordneten Schicht der Trennfolie, die im Wesentlichen aus Polyethylenvinylalkohol-Copolymer gebildet wird und den Außenschichten aus thermoplastischen Polyurethanen Zwischenschichten aus einem Olefin-basierenden Polymer-Haftvermittler befinden.

6. Verwendung nach Anspruch 5, **dadurch gekennzeichnet, dass** das als Polymer-Haflvermittler eingesetzte Material im Wesentlichen aus modifiziertem Ethylenvinylacetat-Copolymer besteht

7. Verwendung nach Ansprüchen 1 bis 5, **dadurch gekennzeichnet, dass** mindestens eine der Schichten der Trennfolie aus einem TPU auf Basis einer Polyether-Diolkomponente besteht.

8. Verwendung nach Ansprüchen 1 bis 7, **dadurch gekennzeichnet, dass** die aus thermoplastischen Polyurethanen gebildeten Schichten der Trennfolie mit Additiven aus der Gruppe umfassend
I. Antiblockmittel, anorganische oder organische Abstandshalter,
II. Gleit- oder Entformungsmittel,
III. Pigmente oder Füllstoffe und
IV. Stabilisatoren
ausgerüstet sind, wobei der Anteil der genannten Additive I bis IV in Summe bevorzugt zwischen 0 Gew.-% und 30 Gew.-% liegt.

9. Verwendung nach Ansprüchen 1 bis 8, **dadurch gekennzeichnet, dass** die Trennfolie einen mindestens dreischichtigen Aufbau aufweist und mindestens eine der Außenschichten bis zu 30 Gew.-% eines Antistatikums enthält.

10. Verwendung nach Ansprüchen 1 bis 9, **dadurch gekennzeichnet, dass** die Trennfolie eine Gesamtdicke zwischen 50 µm und 800 µm hat, wobei die Dicke der Schicht(en) aus thermoplastischen Polyurethanen zwischen 20 µm und 400 µm, die Dicke der Haftvermittler-Schicht(en) zwischen 5 µm und 200 µm und die der Schicht(en) aus Polyethylenvinylalkohol-Copolymer 5 µm bis 50 µm beträgt.

11. Verwendung nach Ansprüchen 1 bis 10, **dadurch gekennzeichnet, dass** die Trennfolie in einem Blasfolien-Coextrusion hergestellt wurde.

12. Verwendung nach Ansprüchen 1 bis 10, **dadurch gekennzeichnet, dass** mindestens eine der Außenschichten der Trennfolie einer dem Stand der Technik entsprechenden physikalischen und/oder chemischen Oberflächenbehandlung unterzogen wurde.

13. Verwendung nach Ansprüchen 1 bis 10, **dadurch gekennzeichnet, dass** die Trennfolie flächig mit mindestens einer weiteren Kunststofffolie und/oder Gewebebahn verbunden ist.

## Claims

1. Use of a multilayer foil with at least two external layers made of thermoplastic polyurethanes (TPUs) and with an internal layer based on polyethylene-vinyl alcohol copolymer as separator foil between gas mixtures of different composition, **characterized in that** the separator foil has been produced in a coextrusion process.

2. Use according to Claim 1, **characterized in that** the separator foil is used as membrane foil in biogas systems.

3. Use according to Claim 1 or 2, **characterized in that** the methane transmission rate of the separator foil, measured in accordance with ISO 15195-1 at 23°C/0% relative humidity, based on a foil thickness of 100 µm, is less than 150 cm³/m²•d•bar.

4. Use according to Claim 1 or 2, **characterized in that** the ammonia transmission rate of the separator foil, measured in accordance with ISO 15195-1 at 23°C/0% relative humidity, based on a foil thickness of 100 µm, is less than 100 cm³/m²•d•bar.

5. Use according to Claim 3 or 4, **characterized in that** between the internally arranged layer of the separator foil which is in essence formed from polyethylene-vinyl alcohol copolymer and the external layers made of thermoplastic polyurethanes there are intermediate layers made of an olefin-based polymer adhesion promoter.

6. Use according to Claim 5, **characterized in that** the material used as polymer adhesion promoter in essence consists of modified ethylene-vinyl acetate copolymer.

7. Use according to Claims 1 to 5, **characterized in that** at least one of the layers of the separator foil is composed of a TPU based on a polyether diol component.

8. Use according to Claims 1 to 7, **characterized in that** the layers formed from thermoplastic polyurethanes in the separator foil have been equipped with additives from the group comprising
I. antiblocking agents, inorganic or organic spacers,
II. lubricants or mould-release agents,
III. pigments or fillers and
IV. stabilizers,
where the proportion of the additives I to IV mentioned is preferably in total from 0% by weight to 30% by weight.

9. Use according to Claims 1 to 8, **characterized in that** the separator foil has an at least three-layer structure and at least one of the external layers comprises up to 30% by weight of an antistatic agent.

10. Use according to Claims 1 to 9, **characterized in that** the total thickness of the separator foil is from 50 µm to 800 µm, where the thickness of the layer(s) made of thermoplastic polyurethanes is from 20 µm to 400 µm, the thickness of the adhesion promoter layer(s) is from 5 µm to 200 µm and the thickness of the layer(s) made of polyethylene-vinyl alcohol copolymer is from 5 µm to 50 µm.

11. Use according to Claims 1 to 10, **characterized in that** the separator foil has been produced in a blown film coextrusion process.

12. Use according to Claims 1 to 10, **characterized in that** at least one of the external layers of the separator foil has been subjected to a physical and/or chemical surface treatment corresponding to the prior art.

13. Use according to Claims 1 to 10, **characterized in that** the separator foil has, at its surface, been bonded to at least one other plastics foil and/or textile web.

## Revendications

1. Utilisation d'une feuille multicouche présentant au moins deux couches externes en polyuréthanes thermoplastiques (TPU) et une couche interne à base d'un copolymère de poly(éthylène-alcool vinylique) comme feuille de séparation entre des mélanges gazeux de composition différente, **caractérisée en ce que** la feuille de séparation a été préparée dans un procédé de coextrusion.

2. Utilisation selon la revendication 1, **caractérisée en ce que** la feuille de séparation est utilisée comme feuille de membrane dans les installations de biogaz.

3. Utilisation selon la revendication 1 ou 2, **caractérisée en ce que** la perméation du méthane de la feuille de séparation est inférieure à 150 cm³/m²*j*bar, mesurée selon la norme ISO 15195-1 à 23°C/0% d'humidité relative, par rapport à une épaisseur de feuille de 100 µm.

4. Utilisation selon la revendication 1 ou 2, **caractérisée en ce que** la perméation d'ammoniac de la feuille de séparation est inférieure à 100 cm³/m²*j*bar, mesurée selon la norme ISO 15195-1 à 23°C/0% d'humidité relative, par rapport à une épaisseur de feuille de 100 µm.

5. Utilisation selon la revendication 3 ou 4, **caractérisée en ce que** des couches intermédiaires en un promoteur d'adhérence en polymère à base d'une oléfine se trouvent entre la couche interne de la feuille de séparation, qui est essentiellement formée de copolymère de poly(éthylène-alcool vinylique) et les couches externes en polyuréthanes thermoplastiques.

6. Utilisation selon la revendication 5, **caractérisée en ce que** le matériau utilisé comme promoteur d'adhérence en polymère est essentiellement constitué par un copolymère modifié d'éthylène-acétate de vinyle.

7. Utilisation selon les revendications 1 à 5, **caractérisée en ce qu'**au moins une des couches de la feuille de séparation est constituée par un TPU à base d'un composant polyétherdiol.

8. Utilisation selon les revendications 1 à 7, **caractérisée en ce que** les couches formées à partir de polyuréthanes thermoplastiques de la feuille de séparation sont apprêtées avec des additifs du groupe comprenant
I. les agents antiadhésifs, les écarteurs inorganiques ou organiques,
II. les agents lubrifiants ou de démoulage,
III. les pigments ou les charges et
IV. les stabilisants
la proportion des additifs mentionnés I à IV, en les additionnant, se situant de préférence entre 0% en poids et 30% en poids.

9. Utilisation selon les revendications 1 à 8, **caractérisée en ce que** la feuille de séparation présente une structure au moins à trois couches et au moins une des couches externes contient jusqu'à 30% en poids d'un agent antistatique.

10. Utilisation selon les revendications 1 à 9, **caractérisée en ce que** la feuille de séparation présente une épaisseur totale entre 50 µm et 800 µm, l'épaisseur de la/des couche(s) en polyuréthanes thermoplastiques valant entre 20 µm et 400 µm, l'épaisseur de la/des couche(s) de promoteur d'adhérence valant entre 5 µm et 200 µm et celle de la/des couche(s) en copolymère de poly(éthylène-alcool vinylique) valant de 5 µm à 50 µm.

11. Utilisation selon les revendications 1 à 10, **caractérisée en ce que** la feuille de séparation a été préparée dans une coextrusion de feuilles soufflées.

12. Utilisation selon les revendications 1 à 10, **caractérisée en ce qu'**au moins une des couches externes de la feuille de séparation est soumise à un traitement de surface physique et/ou chimique correspondant à l'état de la technique.

13. Utilisation selon les revendications 1 à 10, **caractérisée en ce que** la feuille de séparation est reliée en surface à au moins une autre feuille en matériau synthétique et/ou bande en tissu.
